# EUROPEAN PATENT APPLICATION

(11) **EP 4 692 126 A1**
(43) Date of publication of application: **11.02.2026**
(21) Application number: 24784229.7
(22) Date of filing: 01.04.2024
(51) Int. Cl.: C07K 16/30, A61K 39/395

(54) **ANTI-5T4 ANTIBODY AND USE THEREOF**

(30) Priority: 03.04.2023 CN 202310345194
(71) Applicant: Sound Biopharmaceuticals Co. Ltd., Chengdu, Sichuan 610219 (CN)
(72) Inventor: HUANG, Ying, Chengdu, Sichuan 610219 (CN); MA, Fanxin, Chengdu, Sichuan 610219 (CN); LI, Qing, Chengdu, Sichuan 610219 (CN); WEN, Shoubin, Chengdu, Sichuan 610219 (CN); YANG, Zhouning, Chengdu, Sichuan 610219 (CN); LIU, Shuang, Chengdu, Sichuan 610219 (CN); CHEN, Pengyu, Chengdu, Sichuan 610219 (CN); REN, Ziqing, Chengdu, Sichuan 610219 (CN)
(74) Representative: Kuttenkeuler, David
(86) International application number: PCT/CN2024/085274
(87) International publication number: WO 2024/208145

(57) **Abstract**

The present invention relates to anti-5T4 antibodies, particularly humanized antibodies. The present invention also relates to pharmaceutical compositions comprising these antibodies and medical uses thereof. The antibodies have high affinity, good cell binding activity, and high internalization rate, and can be used to treat diseases overexpressing 5T4, such as a cancer.

## Description

### TECHNICAL FIELD

The present invention relates to anti-5T4 antibodies, particularly humanized antibodies. The present invention also relates to pharmaceutical compositions comprising these antibodies and medical uses thereof.

### BACKGROUND

5T4 (trophoblast glycoprotein) was first discovered in embryonic cells 20 years ago. It is a 72 kDa glycoprotein recognized by mouse monoclonal antibody 5T4 through indirect immunofluorescence staining. It is expressed in human trophoblast cells and highly expressed in various tumor tissues, but not expressed or expressed at a low level in normal adult tissues. The 5T4 gene is located on chromosome 6, between the regions of 6q14-q15, and encodes 420 amino acids. The homology of 5T4 protein sequence between humans and mice is as high as 81%. 5T4 protein has a large amount of glycosylation modifications, but its biological function has not been fully studied.

5T4 is overexpressed in some tumors, such as breast cancer, lung cancer and kidney cancer, but it is expressed at very low level in normal tissues. In addition, high expression of 5T4 is positively correlated with poor prognosis in head and neck tumors and colorectal cancer. Recent studies have shown that the proportion of 5T4 positive B cell populations in relapsed patients is higher in lymphocytic leukemia. The expression of 5T4 in cancer stem cells (CSCs) is associated with poor overall prognosis in non-small cell lung cancer, which may be related to tumor metastasis, drug resistance, and recurrence. Under normal circumstances, 5T4 is mainly expressed in embryonic cells to enhance chemotaxis and play a key role in embryonic development and cell differentiation. Pathologically, overexpression of 5T4 is associated with downregulation of E-cadherin, leading to the metastasis and spread of epithelial tumors. 5T4 also participates in regulating Wnt signaling that is independent of β-catenin by modulating LRP6 subcellular localization. In addition, 5T4 regulates the chemotaxis mediated by CXCL12/CXCR4 chemokines, which may promote the spread of 5T4 positive tumor cells.

The first generation of chimeric antigen receptor T cells targeting 5T4 expressed by human T cells showed in vitro experiments that they promote the release of specific cytokines and exhibit certain cytotoxicity towards 5T4 positive cells. This chimeric antigen receptor targeting 5T4 is used to modify T cells derived from renal cell carcinoma (RCC) patients and can effectively kill renal cell carcinoma cells in vitro. In addition, B16 tumor mice with positive 5T4 expression were vaccinated and their survival was prolonged after CAR-T cell therapy; The extension of survival is the result of the synergistic effect of CAR-T cells and vaccines targeting 5T4, which provides evidence for the synergistic effect of their combined use. These research results indicate that 5T4 is a potential tumor treatment target.

### SUMMARY

In a first aspect, the present invention provides a monoclonal antibody binding to 5T4 (trophoblast glycoprotein) or an antigen binding fragment thereof, comprising a light chain variable region and a heavy chain variable region, wherein the light chain variable region comprises LCDR1, LCDR2, and LCDR3, and the heavy chain variable region comprises HCDR1, HCDR2, and HCDR3, wherein: the LCDR1, LCDR2, and LCDR3 comprise or consist of respective CDR1, CDR2, CDR3 of a light chain variable region with an amino acid sequence set forth in SEQ ID NO: 7, SEQ ID NO: 9, SEQ ID NO: 10, SEQ ID NO: 11, or SEQ ID NO: 12, or equivalent variants thereof, and the HCDR1, HCDR2, and HCDR3 comprise or consist of respective CDR1, CDR2, CDR3 of a heavy chain variable region with an amino acid sequence set forth in SEQ ID NO: 8, SEQ ID NO: 13, SEQ ID NO: 14, SEQ ID NO: 15, SEQ ID NO: 16, or SEQ ID NO: 17, or equivalent variants thereof.

In some embodiments, the LCDR1, LCDR2, LCDR3, HCDR1, HCDR2, and HCDR3 are defined according to any one of the definition schemes selected from the group consisting of IMGT, Kabat, Chothia, Contact, or Martin definition scheme.

In some embodiments, the LCDR1 comprises or consists of a sequence set forth in SEQ ID NO: 1 or an equivalent variant thereof, the LCDR2 comprises or consists of a sequence set forth in SEQ ID NO: 2 or an equivalent variant thereof, and the LCDR3 comprises or consists of a sequence set forth in SEQ ID NO: 3 or an equivalent variant thereof, and the HCDR1 comprises or consists of a sequence set forth in SEQ ID NO: 4 or an equivalent variant thereof, the HCDR2 comprises or consists of a sequence set forth in SEQ ID NO: 5 or an equivalent variant thereof, and the HCDR3 comprises or consists of a sequence set forth in SEQ ID NO: 6 or an equivalent variant thereof.

In some embodiments, the light chain variable region comprises or consists of a sequence set forth in SEQ ID NO: 7 or an equivalent variant thereof, and the heavy chain variable region comprises or consists of a sequence set forth in SEQ ID NO: 8 or an equivalent variant thereof.

In some embodiments, the antibody is a humanized antibody or a chimeric antibody.

In some embodiments, the antibody is a humanized antibody. In some embodiments, the light chain variable region comprises or consists of any sequence selected from the group consisting of SEQ ID NO: 9, SEQ ID NO: 10, SEQ ID NO: 11, and SEQ ID NO: 12, and an equivalent variant of each thereof, and the heavy chain variable region comprises or consists of any sequence selected from the group consisting of SEQ ID NO: 13, SEQ ID NO: 14, SEQ ID NO: 15, SEQ ID NO: 16, and SEQ ID NO: 17, and an equivalent variant of each thereof.

In another aspect, the present invention provides a polynucleotide comprising a polynucleotide encoding any one of the antibodies described above. In another aspect, the present invention further provides a vector comprising the polynucleotide described above. In another aspect, the present invention further provides a non-human host cell comprising the vector described above. In addition, the present invention further provides a cell line for generating the antibody of the present invention, and a method for preparing the antibody by culturing an antibody-producing cell line.

In a second aspect, the present invention provides a pharmaceutical composition comprising a therapeutically effective amount of any one of the antibody, polynucleotide, vector, or host cell described above, and a pharmaceutically acceptable carrier.

In a third aspect, the present invention provides use of any one of the antibody, polynucleotide, vector, or host cell described above in the manufacture of a medicament for treating a cancer.

In a fourth aspect, the present invention provides any one of the antibody, polynucleotide, vector, or host cell described above for use in treating a cancer.

In a fifth aspect, the present invention provides a method for treating a cancer, comprising administering to a subject an effective amount of any one of the antibody, polynucleotide, vector, or host cell described above.

In the above related aspects, the cancer comprises but is not limited to bladder cancer, breast cancer, cervical cancer, colorectal cancer, gastric cancer, lung cancer (such as non-small cell lung cancer), mesothelioma, ovarian cancer, pancreatic cancer, prostate cancer, kidney cancer, thyroid cancer, head and neck cancer, lymphoma, leukemia (such as acute myeloid leukemia).

The anti-5T4 monoclonal antibodies of the present invention have high affinity, good cell binding activity, and high internalization rate, and can be used to treat diseases overexpressing 5T4, such as a cancer.

Other aspects and advantages of the present invention are apparent from the following detailed description of the invention.

### BRIEF DESCRIPTION OF THE DRAWINGS

**FIG. 1****:** Internalization rate test of humanized antibodies.
**FIG. 2****:** Measurement of affinity by ELISA.
**FIG. 3****:** Measurement of affinity by Biacore.
**FIG. 4****:** Measurement of cross reactivity by ELISA.
**FIG. 5****:** Measurement of cell binding activity by FACS.
**FIG. 6****:** Measurement of internalization rate by FACS.

### DETAILED DESCRIPTION

### Definitions

In the present invention, "about" means that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Or, "about" or "substantially comprising" may mean a range of up to 20%. Furthermore, particularly for biological systems or processes, the term may mean up to an order of magnitude or up to 5-fold of a numerical value. Unless otherwise specified, when a specific value is provided in the present application and claims, the meaning of "about" or "substantially comprising" should be assumed to be within an acceptable error range for that specific value.

A composition or method described herein as "comprising" one or more named elements or steps is open-ended, meaning that the named elements or steps are essential, but other elements or steps may be added within the scope of the composition or method. It is also understood that any composition or method described as "comprising" one or more named elements or steps also describes the corresponding, more limited composition or method "consisting essentially of" the same named elements or steps, meaning that the composition or method includes the named essential elements or steps and may also include additional elements or steps that do not materially affect the basic and novel characteristic(s) of the composition or method.

As used herein, the term "antibody" refers to any form of antibody that exhibits a desired biological activity (for example inhibiting the binding of a ligand to a receptor thereof, or inhibiting receptor signal transduction induced by a ligand). An "antibody fragment" and an "antigen-binding fragment" refer to an antigen-binding fragment of an antibody and an antibody analogue, which generally include at least a portion of the antigen-binding region or variable region (e.g. one or more CDRs) of a parental antibody. In some embodiments, the antibody is a monoclonal antibody. In some other embodiments, the antibody is a polyclonal antibody.

As used herein, the term "monoclonal antibody" refers to an antibody obtained from a population of substantially homogeneous antibodies, i.e., the individual antibodies that make up the population are identical except for possible natural mutations that may be present in minor amounts. Monoclonal antibodies are highly specific and can be directed against a single antigenic site. Furthermore, in contrast to conventional (polyclonal) antibody preparations, which typically include multiple different antibodies directed against multiple different determinants (epitopes), each monoclonal antibody is directed against only a single determinant on an antigen. The modifier "monoclonal" refers to the properties of an antibody obtained from a substantially homogeneous population of antibodies and should not be construed as requiring any particular method to prepare said antibody. For example, the monoclonal antibodies used for the present invention may be prepared by hybridoma or recombinant DNA.

Monoclonal antibody may include "chimeric" antibody, humanized antibody, or fully humanized antibody. In some embodiments, an antibody consititutes part of a larger biomolecule, such as a fusion protein or an antibody-drug conjugate. An antibody fragment retains at least some of the binding specificity of the parent antibody. Typically, an antibody fragment retains at least 10% of the parental binding activity when that activity is expressed on a molar basis. Preferably, an antibody fragment retains at least 20%, 50%, 70%, 80%, 90%, 95% or 100% or more of the parental antibody's binding affinity for the target.

As used herein, the term "humanized antibody" refers to an antibody that includes CDRs of an antibody derived from a mammal other than human, as well as framework regions (FR) and constant regions of a human antibody.

An "equivalent variant" of an antibody or polypeptide refers to an antibody or polypeptide that has a certain degree of homology or sequence identity with the amino acid sequence of the antibody or polypeptide. In some aspects, the sequence identity is at least about 70%, 75%, 80%, 85%, 90%, 95%, 98%, or 99%. In some aspects, compared to a reference antibody or polypeptide, an equivalent variant thereof has one, two, three, four, or five additions, deletions, substitutions, and combinations thereof. In some aspects, an equivalent variant of an antibody or polypeptide retains the activity (e.g. epitope binding) or structure (e.g. salt bridge) of the reference sequence.

As used herein, a "variant" sequence refers to a sequence that differs from the sequence shown at one or more amino acid residues but retains the biological activity of the resulting molecule.

As used herein, "% identity" between two sequences refers to a function of the number of identical positions shared by the sequences, i.e., % identity = number of equivalent positions/total number of positions x 100, wherein the number of gaps and the length of each gap will be considered, and the gaps need to be introduced when performing an optimal alignment of the two sequences. Sequences alignment and determination of % identity between two sequences may be accomplished using mathematical algorithms.

"Conservative substitution" refers to substitutions of amino acids known to those of skill in this art and may be made generally without altering the biological activity of the resulting molecule. Those of skill in this art recognize that, in general, single amino acid substitutions in non-essential regions of a polypeptide do not alter or substantially alter biological activity. "Do not alter or substantially alter" refers to having a difference of no more than about 20%, about 15%, about 10%, about 9%, about 8%, about 7%, about 6%, about 5%, about 4%, about 3%, about 2%, or about 1% in one or more aspects compared to the object being compared when measured using the same or similar methods.

Generally, CDR3 is considered to play a more important role in antigen recognition than other CDRs. Therefore, in the case of substitution, the present invention prefers conservative substitution of CDRs other than CDR3. In some embodiments, CDR3 is not substituted. Preferred amino acid substitutions include but are not limited to those that: (1) reduce susceptibility to proteolysis, (2) reduce susceptibility to oxidation, (3) alter binding affinity for forming protein complexes, and (4) confer or modify other physicochemical or functional properties of such analogs. Analogs can include various muteins of a sequence other than the naturally-occurring peptide sequence. For example, single or multiple amino acid substitutions (preferably conservative amino acid substitutions) can be made in the naturally-occurring sequence (preferably in the portion of the polypeptide outside the domain(s) forming intermolecular contacts). A conservative amino acid substitution should not substantially change the structural characteristics of the parent sequence (e.g., a replacement amino acid should not tend to break a helix that occurs in the parent sequence, or disrupt other types of secondary structure that characterizes the parent sequence).

It is expected that the binding domain of the antibody or an antigen binding fragment thereof disclosed herein may carry a signal peptide, which is usually located at the N-terminus of the secreted protein and generally consists of 15-30 amino acids. When the signal peptide sequence is synthesized, it is recognized by the signal recognition granule (SRP), protein synthesis is suspended or slowed down, the signal recognition granule carries the ribosome to the endoplasmic reticulum, and the protein synthesis restarts. Under the guidance of the signal peptide, the newly synthesized protein enters the endoplasmic reticulum cavity, and the signal peptide sequence is cleaved under the action of the signal peptidase. If the termination transit sequence exists at the C-terminus of the nascent peptide chain, it may not be cleaved by the signal peptidase, for example, ovalbumin contains an internal signal peptide, and neither its precursor nor the mature form is cleaved by signal peptidase.

According to the present invention, the term "binding" preferably refers to specific binding. When referring to a ligand/receptor, an antibody/antigen or other binding pairs, "specific" binding refers to determining whether there is a binding reaction of the protein in a heterogeneous population of proteins and/or other biochemical reagents. Therefore, under the specified conditions, a specific ligand/antigen binds to a specific receptor/antibody, and does not bind to other proteins present in a sample in a significant amount. "Specifically binds" means that the monoclonal antibody or an antigen binding fragment thereof disclosed herein is capable of specifically interacting with at least two, three, four, five, six, seven, eight or more amino acids of each human target molecule. The "specific binding" of an antibody is mainly characterized by two parameters: a qualitative parameter (binding epitope or antibody binding site) and a quantitative parameter (binding affinity or binding strength). Antibody binding epitopes may be determined by FACS, peptide dot epitope mapping, mass spectrometry, or peptide ELISA. The Biacore and/or ELISA may measure the binding strength of an antibody to a specific epitope. Signal-to-noise ratios are often calculated as a representative measure of binding specificity. In such a signal-to-noise ratio, the signal represents the strength of antibody binding to the target epitope, and the noise represents the strength of antibody binding to other non-target epitopes. Preferably, when the signal-to-noise ratio for a target epitope is about 50, the evaluated antibody may be considered to bind to the target epitope in a specific manner, i.e., "specifically binds". An antigen-binding protein (including an antibody) "specifically binds" to an antigen if it binds to the antigen with high binding affinity as determined by a value of a dissociation constant (KD). In some embodiments, the affinity constant KD is lower than 10⁻⁹ M. As used herein, the term "KD" refers to an equilibrium dissociation constant of a specific antibody-antigen interaction.

Humanized antibodies are antibody molecules derived from a non-human species antibody that bind the desired antigen having one or more complementarity determining regions (CDRs) from the non-human species and framework regions from a human immunoglobulin molecule. Often, framework residues in the human framework regions will be substituted with the corresponding residue from the CDR donor antibody to alter, preferably improve, antigen-binding. These framework substitutions are identified by methods well known in the art, e.g., by modeling of the interactions of the CDR and framework residues to identify framework residues important for antigen-binding and sequence comparison to identify unusual framework residues at particular positions. Antibodies can be humanized using a variety of techniques known in the art including, for example, CDR-grafting, veneering or resurfacing, and chain shuffling, ect.

Completely human antibodies are particularly desirable for therapeutic treatment of human patients. Human antibodies can be made by a variety of methods known in the art including phage display methods using antibody libraries derived from human immunoglobulin sequences.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring that express human antibodies. The transgenic mice are immunized in the normal fashion with a selected antigen, e.g., all or a portion of a desired target polypeptide. Monoclonal antibodies directed against the antigen can be obtained from the immunized, transgenic mice using conventional hybridoma technology. The human immunoglobulin transgenes harbored by the transgenic mice rearrange during B-cell differentiation, and subsequently undergo class switching and somatic mutation. Thus, using such a technique, it is possible to produce therapeutically useful IgG, IgA, IgM and IgE antibodies.

Completely human antibodies which recognize a selected epitope can also be generated using a technique referred to as "guided selection". In this approach a selected non-human monoclonal antibody, e.g., a mouse antibody, is used to guide the selection of a completely human antibody recognizing the same epitope.

DNA encoding desired monoclonal antibodies can be readily isolated and sequenced using conventional procedures (e.g., by using oligonucleotide probes that are capable of binding specifically to genes encoding the heavy and light chains of murine antibodies). The isolated and subcloned hybridoma cells serve as a preferred source of such DNA. Once isolated, the DNA may be placed into expression vectors, which are then transfected into prokaryotic or eukaryotic host cells such as E. coli cells, simian COS cells, Chinese Hamster Ovary (CHO) cells or myeloma cells that do not otherwise produce immunoglobulins. More particularly, the isolated DNA may be used to clone constant and variable region sequences for the manufacture of antibodies. Essentially, this entails extraction of RNA from the selected cells, conversion to cDNA, and amplification by PCR using Ig specific primers. As described herein, transformed cells expressing the desired antibody can be grown up in relatively large quantities to provide clinical and commercial supplies of the immunoglobulin.

Additionally, using routine recombinant DNA techniques, one or more of the CDRs of the antigen-binding polypeptides of the present disclosure, may be inserted within framework regions, e.g., into human framework regions to humanize a non-human antibody. The framework regions may be naturally occurring or consensus framework regions, and preferably human framework regions. Preferably, the polynucleotide generated by the combination of the framework regions and CDRs encodes an antibody that specifically binds to at least one epitope of a desired polypeptide, e.g., LIGHT. Preferably, one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present disclosure and within the skill of the art.

As used herein, the term "patient" or "subject" refers to any organism to which a provided antibody, derivative thereof, or composition is or may be administered, for experimental, diagnostic, prophylactic, cosmetic, or therapeutic purposes. Typical subjects include animals (e.g, mammals such as mice, rats, rabbits, non-human primates, and/or humans). In some embodiments, a subject is a human. In some embodiments, a subject is suffering from or susceptible to one or more disorders or conditions. A patient may display one or more symptoms of a disorder or condition, or may have been diagnosed with one or more disorders or conditions. In some embodiments, a patient is receiving or has received certain therapy to diagnose and/or to treat such disease, disorder, or condition.

The term "treating" or "treatment" as used herein refers to a therapeutic and preventive means in which the occurrence or progression of an undesired pathological change or disorder is prevented from or slowed in a subject. Beneficial or desirable clinical outcomes include, but are not limited to, alleviation of symptoms, reduction in disease severity, stabilization of disease states (i.e., no deterioration), delay or slowing of disease progression, reduction or mitigation of disease states, and local or holistic cure of disease, whether or not these results are detectable. "Treatment" can also refer to prolonged survival as compared to the expected survival without treatment. Subjects in need of treatment include those already having the disease or condition as well as those who are prone to have the disease or condition or who are in need of prevention from the disease or condition.

When "administering" and "treating" or "preventing" refer to an animal, human, experimental subject, cell, tissue, organ or biological fluid, it refers to contacting the animal, human, subject, cell, tissue, organ or biological fluid with an exogenous drug, therapeutic agent, diagnostic agent or composition. "Administration" and "treatment" may refer to, for example, therapeutic methods, pharmacokinetic methods, diagnostic methods, research methods, and experimental methods. Treatment of the cells includes contacting the agent with the cells and contacting the agent with a fluid, wherein the fluid is in contact with the cells. "Administration" and "treatment" also mean *in vitro* and *ex vivo* treatment of cells, e.g., by agents, diagnostic agents, binding compositions, or by other cells.

As used herein, the term "therapeutically effective amount" or "effective amount" refers to when the antibody or derivative thereof disclosed herein is administered alone or in combination with another therapeutic agent to a cell, tissue or subject, it effectively prevents or slows the amount of the disease or condition to be treated. A therapeutically effective dose further refers to the amount sufficient to cause alleviation of symptoms, such as treating, curing, preventing or alleviating related medical conditions, or improving the treatment rate, cure rate, prevention rate, or alleviation rate of the symptoms. When an active ingredient alone is administered to an individual, the therapeutically effective amount refers to the mount of the alone ingredient. When a combination is administered, the therapeutically effective amount refers to the combined amount of active ingredients that produce a therapeutic effect, regardless of whether it is administered in combination, sequentially or simultaneously. A therapeutically effective amount will reduce symptoms usually by at least 10%; usually at least 20%; preferably at least about 30%; more preferably at least 40% and most preferably at least 50%.

As used herein, "pharmaceutically acceptable carrier" includes a material which, when combined with an active ingredient of a composition, allows the ingredient to retain biological activity and without causing disruptive reactions with the subject's immune system. Such may include stabilizers, preservatives, salt or sugar complexes or crystals, and the like. "Pharmaceutically acceptable" refers to molecules and ingredients that will not produce allergic reactions or similar undesirable reactions when applied to human body.

### Anti-5T4 Antibody

The inventors obtained monoclonal antibodies by immunizing mice with 5T4 antigen and screening, and then obtained humanized anti-5T4 antibodies by performing humanization modification to the antibodies.

In an aspect, the present invention provides a monoclonal antibody binding to 5T4 (trophoblast glycoprotein) or an antigen binding fragment thereof, comprising a light chain variable region and a heavy chain variable region, wherein the light chain variable region comprises LCDR1, LCDR2, and LCDR3, and the heavy chain variable region comprises HCDR1, HCDR2, and HCDR3, wherein: the LCDR1, LCDR2, and LCDR3 comprise or consist of respective CDR1, CDR2, CDR3 of a light chain variable region with an amino acid sequence set forth in SEQ ID NO: 7, SEQ ID NO: 9, SEQ ID NO: 10, SEQ ID NO: 11, or SEQ ID NO: 12, or equivalent variants thereof, and the HCDR1, HCDR2, and HCDR3 comprise or consist of respective CDR1, CDR2, CDR3 of a heavy chain variable region with an amino acid sequence set forth in SEQ ID NO: 8, SEQ ID NO: 13, SEQ ID NO: 14, SEQ ID NO: 15, SEQ ID NO: 16, or SEQ ID NO: 17, or equivalent variants thereof.

In some embodiments, the CDR1, CDR1, and CDR3 (for example, LCDR1, LCDR2, LCDR3, HCDR1, HCDR2, and HCDR3) are defined according to any one of the definition schemes selected from the group consisting of IMGT, Kabat, Chothia, Contact, or Martin definition scheme. In some embodiments, the CDR1, CDR1, and CDR3 (for example, LCDR1, LCDR2, LCDR3, HCDR1, HCDR2, and HCDR3) are defined according to the IMGT definition scheme.

In some embodiments, the LCDR1 comprises or consists of a sequence set forth in SEQ ID NO: 1 or an equivalent variant thereof, the LCDR2 comprises or consists of a sequence set forth in SEQ ID NO: 2 or an equivalent variant thereof, and the LCDR3 comprises or consists of a sequence set forth in SEQ ID NO: 3 or an equivalent variant thereof, and the HCDR1 comprises or consists of a sequence set forth in SEQ ID NO: 4 or an equivalent variant thereof, the HCDR2 comprises or consists of a sequence set forth in SEQ ID NO: 5 or an equivalent variant thereof, and the HCDR3 comprises or consists of a sequence set forth in SEQ ID NO: 6 or an equivalent variant thereof.

In some embodiments, the light chain variable region comprises or consists of a sequence set forth in SEQ ID NO: 7 or an equivalent variant thereof, and the heavy chain variable region comprises or consists of a sequence set forth in SEQ ID NO: 8 or an equivalent variant thereof.

In some embodiments, the antibody is a humanized antibody or a chimeric antibody.

In some embodiments, the antibody is a humanized antibody. In some embodiments, the light chain variable region comprises or consists of any sequence selected from the group consisting of SEQ ID NO: 9, SEQ ID NO: 10, SEQ ID NO: 11, and SEQ ID NO: 12, and an equivalent variant of each thereof, and the heavy chain variable region comprises or consists of any sequence selected from the group consisting of SEQ ID NO: 13, SEQ ID NO: 14, SEQ ID NO: 15, SEQ ID NO: 16, and SEQ ID NO: 17, and an equivalent variant of each thereof.

In some embodiments, the light chain variable region comprises or consists of a sequence set forth in SEQ ID NO: 9 or an equivalent variant thereof, and the heavy chain variable region comprises or consists of a sequence set forth in SEQ ID NO: 13 or an equivalent variant thereof.

In some embodiments, the light chain variable region comprises or consists of a sequence set forth in SEQ ID NO: 10 or an equivalent variant thereof, and the heavy chain variable region comprises or consists of a sequence set forth in SEQ ID NO: 13 or an equivalent variant thereof.

In some embodiments, the light chain variable region comprises or consists of a sequence set forth in SEQ ID NO: 11 or an equivalent variant thereof, and the heavy chain variable region comprises or consists of a sequence set forth in SEQ ID NO: 13 or an equivalent variant thereof.

In some embodiments, the light chain variable region comprises or consists of a sequence set forth in SEQ ID NO: 12 or an equivalent variant thereof, and the heavy chain variable region comprises or consists of a sequence set forth in SEQ ID NO: 13 or an equivalent variant thereof.

In some embodiments, the light chain variable region comprises or consists of a sequence set forth in SEQ ID NO: 9 or an equivalent variant thereof, and the heavy chain variable region comprises or consists of a sequence set forth in SEQ ID NO: 14 or an equivalent variant thereof.

In some embodiments, the light chain variable region comprises or consists of a sequence set forth in SEQ ID NO: 10 or an equivalent variant thereof, and the heavy chain variable region comprises or consists of a sequence set forth in SEQ ID NO: 14 or an equivalent variant thereof.

In some embodiments, the light chain variable region comprises or consists of a sequence set forth in SEQ ID NO: 11 or an equivalent variant thereof, and the heavy chain variable region comprises or consists of a sequence set forth in SEQ ID NO: 14 or an equivalent variant thereof.

In some embodiments, the light chain variable region comprises or consists of a sequence set forth in SEQ ID NO: 12 or an equivalent variant thereof, and the heavy chain variable region comprises or consists of a sequence set forth in SEQ ID NO: 14 or an equivalent variant thereof.

In some embodiments, the light chain variable region comprises or consists of a sequence set forth in SEQ ID NO: 9 or an equivalent variant thereof, and the heavy chain variable region comprises or consists of a sequence set forth in SEQ ID NO: 15 or an equivalent variant thereof.

In some embodiments, the light chain variable region comprises or consists of a sequence set forth in SEQ ID NO: 10 or an equivalent variant thereof, and the heavy chain variable region comprises or consists of a sequence set forth in SEQ ID NO: 15 or an equivalent variant thereof.

In some embodiments, the light chain variable region comprises or consists of a sequence set forth in SEQ ID NO: 11 or an equivalent variant thereof, and the heavy chain variable region comprises or consists of a sequence set forth in SEQ ID NO: 15 or an equivalent variant thereof.

In some embodiments, the light chain variable region comprises or consists of a sequence set forth in SEQ ID NO: 12 or an equivalent variant thereof, and the heavy chain variable region comprises or consists of a sequence set forth in SEQ ID NO: 15 or an equivalent variant thereof.

In some embodiments, the light chain variable region comprises or consists of a sequence set forth in SEQ ID NO: 9 or an equivalent variant thereof, and the heavy chain variable region comprises or consists of a sequence set forth in SEQ ID NO: 16 or an equivalent variant thereof.

In some embodiments, the light chain variable region comprises or consists of a sequence set forth in SEQ ID NO: 10 or an equivalent variant thereof, and the heavy chain variable region comprises or consists of a sequence set forth in SEQ ID NO: 16 or an equivalent variant thereof.

In some embodiments, the light chain variable region comprises or consists of a sequence set forth in SEQ ID NO: 11 or an equivalent variant thereof, and the heavy chain variable region comprises or consists of a sequence set forth in SEQ ID NO: 16 or an equivalent variant thereof.

In some embodiments, the light chain variable region comprises or consists of a sequence set forth in SEQ ID NO: 12 or an equivalent variant thereof, and the heavy chain variable region comprises or consists of a sequence set forth in SEQ ID NO: 16 or an equivalent variant thereof.

In some embodiments, the light chain variable region comprises or consists of a sequence set forth in SEQ ID NO: 9 or an equivalent variant thereof, and the heavy chain variable region comprises or consists of a sequence set forth in SEQ ID NO: 17 or an equivalent variant thereof.

In some embodiments, the light chain variable region comprises or consists of a sequence set forth in SEQ ID NO: 10 or an equivalent variant thereof, and the heavy chain variable region comprises or consists of a sequence set forth in SEQ ID NO: 17 or an equivalent variant thereof.

In some embodiments, the light chain variable region comprises or consists of a sequence set forth in SEQ ID NO: 11 or an equivalent variant thereof, and the heavy chain variable region comprises or consists of a sequence set forth in SEQ ID NO: 17 or an equivalent variant thereof.

In some embodiments, the light chain variable region comprises or consists of a sequence set forth in SEQ ID NO: 12 or an equivalent variant thereof, and the heavy chain variable region comprises or consists of a sequence set forth in SEQ ID NO: 17 or an equivalent variant thereof.

The humanized antibody mentioned above may comprise a light chain constant region and a heavy chain constant region, wherein the light chain constant region is, for example, the human Ig kappa light chain constant region set forth in SEQ ID NO: 18, and the heavy chain constant region is, for example, the human IgG1 heavy chain constant region set forth in SEQ ID NO: 19.

In some embodiments, the equivalent variant of each of the CDR1, CDR1, and CDR3 (for example, LCDR1, LCDR2, LCDR3, HCDR1, HCDR2, and HCDR3) refers to one that has a single amino acid substitution, deletion or insertion compared to the reference sequence.

In some embodiments, the equivalent variant of a variable region refers to one that has at least 75%, 80%, 85%, 90%, 95%, 98%, or 99% sequence identity with the sequence of any one of SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, and SEQ ID NO: 17, and has the same or equivalent CDR1, CDR2 and CDR3.

In some embodiments, the LCDR1 comprises a sequence set forth in SEQ ID NO: 1, the LCDR2 comprises a sequence set forth in SEQ ID NO: 2, the LCDR3 comprises a sequence set forth in SEQ ID NO: 3, and the HCDR1 comprises a sequence set forth in SEQ ID NO: 4, the HCDR2 comprises a sequence set forth in SEQ ID NO: 5, and the HCDR3 comprises a sequence set forth in SEQ ID NO: 6.

In some embodiments, the light chain variable region comprises a sequence set forth in SEQ ID NO: 7, and the heavy chain variable region comprises a sequence set forth in SEQ ID NO: 8.

In some embodiments, the antibody is a humanized antibody. In some embodiments, the light chain variable region comprises any sequence selected from the group consisting of SEQ ID NO: 9, SEQ ID NO: 10, SEQ ID NO: 11, and SEQ ID NO: 12, and the heavy chain variable region comprises any sequence selected from the group consisting of SEQ ID NO: 13, SEQ ID NO: 14, SEQ ID NO: 15, SEQ ID NO: 16, and SEQ ID NO: 17.

In some embodiments, the LCDR1 consists of a sequence set forth in SEQ ID NO: 1, the LCDR2 consists of a sequence set forth in SEQ ID NO: 2, the LCDR3 consists of a sequence set forth in SEQ ID NO: 3, and the HCDR1 consists of a sequence set forth in SEQ ID NO: 4, the HCDR2 consists of a sequence set forth in SEQ ID NO: 5, and the HCDR3 consists of a sequence set forth in SEQ ID NO: 6.

In some embodiments, the light chain variable region consists of a sequence set forth in SEQ ID NO: 7, and the heavy chain variable region consists of a sequence set forth in SEQ ID NO: 8.

In some embodiments, the antibody is a humanized antibody. In some embodiments, the light chain variable region consists of any sequence selected from the group consisting of SEQ ID NO: 9, SEQ ID NO: 10, SEQ ID NO: 11, and SEQ ID NO: 12, and the heavy chain variable region consists of any sequence selected from the group consisting of SEQ ID NO: 13, SEQ ID NO: 14, SEQ ID NO: 15, SEQ ID NO: 16, and SEQ ID NO: 17.

In some embodiments, the antibody is of an IgG type, such as IgG1, IgG2, IgG3, or IgG4 type. In some embodiments, the antibody is of the IgG1 type.

In some embodiments, the substitutions disclosed herein are conservative substitutions.

A "conservative amino acid substitution" is one in which the amino acid residue is replaced with an amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a nonessential amino acid residue in an immunoglobulin polypeptide is preferably replaced with another amino acid residue from the same side chain family. In another embodiment, a string of amino acids can be replaced with a structurally similar string that differs in order and/or composition of side chain family members.

It will also be understood by one of skill in the art that antibodies as disclosed herein may be modified such that they vary in amino acid sequence from the naturally occurring binding polypeptide from which they were derived. For example, a polypeptide or amino acid sequence derived from a designated protein may be similar, e.g., have a certain percent identity to the starting sequence, e.g., it may be 60%, 70%, 75%, 80%, 85%, 90%, 95%, 98%, 99%, or a range between any two of these values, identical to the starting sequence.

In some embodiments, the antibody comprises an amino acid sequence or one or more moieties not normally associated with an antibody. Exemplary modifications are described in more detail herein. For example, an antibody disclosed herein may comprise a flexible linker sequence, or may be modified to add a functional moiety (e.g., polyethylene glycol (PEG), a drug, a toxin, or a label).

Antibodies, variants, or derivatives thereof of the disclosure include derivatives that are modified, i.e., by the covalent attachment of any type of molecule to the antibody such that covalent attachment does not prevent the antibody from binding to the epitope. For example, but not by way of limitation, the antibodies can be modified, e.g., by glycosylation, acetylation, pegylation, phosphorylation, phosphorylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the antibodies may contain one or more non-classical amino acids.

In some embodiments, the antibodies may be conjugated to therapeutic agents, prodrugs, peptides, proteins, enzymes, viruses, lipids, biological response modifiers, pharmaceutical agents, or PEG.

The antibodies may be conjugated or fused to a therapeutic agent, which may include detectable labels such as radioactive labels, an immunomodulator, a hormone, an enzyme, an oligonucleotide, a photoactive therapeutic or diagnostic agent, a cytotoxic agent, which may be a drug or a toxin, an ultrasound enhancing agent, a non-radioactive label, a combination thereof and other such agents known in the art.

The antibodies can be detectably labeled by coupling it to a chemiluminescent compound. The presence of the chemiluminescent-tagged antigen-binding polypeptide is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

The antibodies can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA). Techniques for conjugating various moieties to an antibody are well known.

In another aspect, the present invention provides a polynucleotide comprising a polynucleotide encoding any one of the antibodies of any of the above aspects. In another aspect, the present invention further provides a vector comprising the polynucleotide described above. In another aspect, the present invention further provides a non-human host cell comprising the vector described above.

### Pharmaceutical Composition

In another aspect, the present invention provides a pharmaceutical composition comprising a therapeutically effective amount of any one of the antibody, polynucleotide, vector, or host cell described above, and a pharmaceutically acceptable carrier.

In any of the above aspects, the pharmaceutical composition is for use in treating a disease overexpressing 5T4, such as a cancer. In some embodiments, the cancer comprises but is not limited to bladder cancer, breast cancer, cervical cancer, colorectal cancer, gastric cancer, lung cancer (such as non-small cell lung cancer), mesothelioma, ovarian cancer, pancreatic cancer, prostate cancer, kidney cancer, thyroid cancer, head and neck cancer, lymphoma, leukemia (such as acute myeloid leukemia).

To prepare a pharmaceutical or sterile composition, the drug is mixed with a pharmaceutically acceptable carrier or excipient. Formulations in the form of such as freeze-dried powder, slurry, aqueous solution, or suspension can be prepared by mixing with physiologically acceptable carriers, excipients, or stabilizers. Pharmaceutically acceptable carriers are well-known in the art. It is known in the art how to prepare aqueous compositions containing active ingredients. Generally, these compositions are prepared into injections or sprays, such as liquid solutions or suspensions; It can also be prepared into a solid form suitable for preparation into a solution or suspension before injection or spray.

### Method and Use

In another aspect, the present invention provides a method for treating a cancer, comprising administering to a subject an effective amount of any one of the antibody, polynucleotide, vector, or host cell described above.

Accordingly, in another aspect, the present invention provides use of any one of the antibody, polynucleotide, vector, or host cell described above in the manufacture of a medicament for treating a cancer.

Accordingly, in another aspect, the present invention provides any one of the antibody, polynucleotide, vector, or host cell described above for use in treating a cancer.

In some embodiments, the cancer comprises but is not limited to bladder cancer, breast cancer, cervical cancer, colorectal cancer, gastric cancer, lung cancer (such as non-small cell lung cancer), mesothelioma, ovarian cancer, pancreatic cancer, prostate cancer, kidney cancer, thyroid cancer, head and neck cancer, lymphoma, leukemia (such as acute myeloid leukemia).

Suitable routes of administration include parenteral administration (for example, intramuscular, intravenous or subcutaneous administration) and oral administration. Other conventional administration methods include administration by tracheal intubation, oral ingestion, inhalation, topical application or transdermal, subcutaneous, intraperitoneal, intraarterial.

The appropriate dose is determined by the clinician, for example, using parameters or factors known or suspected to affect the treatment or expected to affect the treatment in the art. Generally, the starting dose is slightly lower than the optimal dose, and thereafter a small increase until the desired or optimal effect relative to any adverse side effects is achieved. Important diagnostic measures include measuring, for example, inflammatory symptoms or the level of inflammatory cytokines produced.

### Sequence Listing

| SEQ ID NO: | Description | Sequence |
|---|---|---|
| 1 | LCDR1 | QDISNY |
| 2 | LCDR2 | HTS |
| 3 | LCDR3 | QQGDTVPLT |
| 4 | HCDR1 | GFTFRNYA |
| 5 | HCDR2 | ISTGGSYT |
| 6 | HCDR3 | AFYYYGSSYSMDY |
| 7 | B5-VL | DIQMTQTTSSLSASLGDRVTISCRASQDISNYLNWYQQKPDG TVKLLIYHTSILHSGVPSRFSGSGSGTDYSLTISNLEQEDIATYF CQQGDTVPLTFGAGTKLELK |
| 8 | B5-VH | EVQLVESGGGLVKPGGSLKLSCAASGFTFRNYAMSWVRQSP DKRLEWVAEISTGGSYTYYPDTVTGRFTISRDNAKNTLYLEM SSLRSEDTAMYYCAFYYYGSSYSMDYWGQGTSVTVSS |
| 9 | hB5-VL1 | DIQMTQSPSSLSASVGDRVTISCRASQDISNYLNWYQQKPGK APKLLIYHTSILHSGVPSRFSGSGSGTDYSLTISSLQPEDIATYF CQQGDTVPLTFGGGTKVEIK |
| 10 | hB5-VL2 | DIQMTQSPSSLSASVGDRVTISCRASQDISNYLNWYQQKPGK VPKLLIYHTSILHSGVPSRFSGSGSGTDYSLTISSLQPEDVATYF CQQGDTVPLTFGGGTKVEIK |
| 11 | hB5-VL3 | DIQMTQTTSSLSASVGDRVTISCRASQDISNYLNWFQQKPGK APKSLIYHTSILHSGVPSKFSGSGSGTDYSLTISSLQPEDFATYF CQQGDTVPLTFGGGTKVEIK |
| 12 | hB5-VL4 | DIQMTQSPSSLSASVGDRVTISCRASQDISNYLNWYQQKPGK APKLLIYHTSILHSGVPSRFSGSGSGTDYSLTISSLQPEDFATYF CQQGDTVPLTFGGGTKVEIK |
| 13 | hB5-VH1 | EVQLVESGGGLVQPGGSLRLSCAASGFTFRNYAMSWVRQAP GKGLEWVAEISTGGSYTYYPDTVTGRFTISRDNAKNSLYLQM NSLRAEDTAVYYCAFYYYGSSYSMDYWGQGTLVTVSS |
| 14 | hB5-VH2 | EVQLVESGGGLVQPGGSLRLSCAASGFTFRNYAMSWVRQAP GKGLEWVAEISTGGSYTYYPDTVTGRFTISRDNSKNTLYLQM NSLRAEDTAVYYCAFYYYGSSYSMDYWGQGTLVTVSS |
| 15 | hB5-VH3 | EVQLVESGGGLVQPGGSLRLSCAASGFTFRNYAMSWVRQAP GKGLEWVAEISTGGSYTYYPDTVTGRFTISRDNAKNTLYLQM NSLRAEDTAVYYCAFYYYGSSYSMDYWGQGTLVTVSS |
| 16 | hB5-VH4 | EVQLVESGGGVVQPGRSLRLSCAASGFTFRNYAMSWVRQAP GKGLEWVAEISTGGSYTYYPDTVTGRFTISRDNSKNTLYLQM SSLRAEDTAVYYCAFYYYGSSYSMDYWGQGTLVTVSS |
| 17 | hB5-VH5 | EVQLVESGGGVVQPGRSLRLSCAASGFTFRNYAMSWVRQAP GKGLEWVAEISTGGSYTYYPDTVTGRFTISRDNAKNTLYLQM NSLRAEDTAVYYCAFYYYGSSYSMDYWGQGTLVTVSS |
| 18 | human Ig kappa light chain constant region (Uniprot: P01834) | |
| 19 | human IgG1 heavy chain constant region (Uniprot: P01857) | |

### EXAMPLES

### Example 1: Screening and sequencing of antibodies

Step 1: CHO cells were infected with lentivirus containing the 5T4 gene, and 6-week-old BALB/c mice were immunized with the constructed CHO-5T4⁺ cells.

Step 2: Cells were fused, and multiple primary cell lines were obtained;

Step 3: The hybridoma containing supernatant secreted by the primary cell lines was screened using ELISA, flow cytometry, and Biacore, and multiple candidate hybridoma clones with good affinity were obtained;

Step 4: The candidate cell lines were subclonized and screened for monoclonal antibodies with high affinity, high internalization rate, and good specificity through ELISA, flow cytometry, SPR, and Western blot.

A candidate monoclonal antibody was screened out using the above method and then named B5. The amino acid sequences of the light and heavy chain variable regions of antibody B5 were obtained using PCR technology, which are SEQ ID NO: 7 and SEQ ID NO: 8, respectively.

### Example 2: Affinity test of antibody B5

The affinity of antibody B5 screened out in Example 1 was measured using Biacore assay.

The specific measurement method was as follows:
1) Hybridoma affinity screening: 5T4 EDC-Fc (molecular weight 65-120 kD) was used as an antigen. A direct method was utilized in which the antigen was coupled to CM5 via amino groups;
2) According to Rmax= (MW_{analyte}/MW_{ligand}) × RL × Sm, the actual coupling amount was 1.5 times that of RL. The coupling amount of the antigen simultaneously appropriate for detection was calculated to be 120 Ru;
3) Coupling: 1 × HBS-EP buffer was used as the coupling buffer, and 50 mM NaOH was used as the washing solution. The antigen was formulated with a pH 4.5 sodium acetate solution to a concentration of 5 µg/mL. Amino coupling was selected, 120 Ru was inputted in the Target level, and the antigen sample, NaOH, EDC, NSH, ethanolamine and empty test tubes were placed in the corresponding positions on the sample tray according to the indicated order;
4) Exploration of regeneration conditions: Glycine hydrochloric acid with a pH of 1.7 was ultimately selected for 10 seconds of regeneration;
5) Sample preparation: For positive control antibody, B5 antibody was diluted with 1 × HBS-EP buffer to 8 nM, 4 nM, 2 nM, 1 nM, 0.5 nM, 0.25 nM, and then centrifugation was performed at 13,000 rpm for 5 minutes; All samples were provided with well replicates; Preparation of hybridoma containing supernatant samples: hybridoma cell containing supernatant was collected, and centrifugation was performed at 13000 rpm for 5 minutes, followed by dilution twice with 1 × HBS-EP buffer, in which each sample was provided with well replicates;
6) Program settings: 2-1 was selected for the Flow pat in the Kinetics/Affinity option; 2 was selected for the Regeneration; Contact time was 180 s; Dissociation time was set to 600 s; Regeneration conditions: Gly 1.7, 10 s; The samples for repeated testing was spaced apart, and three 0 concentrations and three start up was set, and start was clicked to run the program;
7) Results analysis: Appropriate concentration was selected to correspond to the response unit changing curve, a 1:2 calculation model was used for fitting, and kinetic analysis was conducted.

The measuring results are shown in Table 1.

**Table 1. Affinity measurement of antibody B5**

| Sample Name | Ka(1/Ms) | Kd(1/s) | KD(M) |
|---|---|---|---|
| B5 | 8.03E+05 | 1.45E-05 | 1.8 E-11 |

### Example 3: Construction of humanized antibodies

The antibody B5 screened out in Example 1 was subjected to humanization modification, and four humanized light chain variable region sequences hB5-VL1 (SEQ ID NO: 9), hB5-VL2 (SEQ ID NO: 10), hB5-VL3 (SEQ ID NO: 11), hB5-VL4 (SEQ ID NO: 12), and 5 humanized heavy chain variable region sequences hB5-VH1 (SEQ ID NO: 13), hB5-VH2 (SEQ ID NO: 14), hB5-VH3 (SEQ ID NO: 15), hB5-VH4 (SEQ ID NO: 16), hB5-VH5 (SEQ ID NO: 17), were obtained.

The above light chain variable regions were combined with the above heavy chain variable regions, and 20 humanized antibodies as shown in Table 2 were constructed, which were named hB5-2-1~hB5-2-20 respectively. The constant regions of these antibodies were all human Ig kappa light chain constant region (SEQ ID NO: 18) and human IgG1 heavy chain constant region (SEQ ID NO: 19).

**Table 2. Construction of humanized antibodies**

| | light chain hB5-VL1 | light chain hB5-VL2 | light chain hB5-VL3 | light chain hB5-VL4 |
|---|---|---|---|---|
| heavy chain hB5-VH1 | hB5-2-1 | hB5-2-2 | hB5-2-3 | hB5-2-4 |
| heavy chain hB5-VH2 | hB5-2-5 | hB5-2-6 | hB5-2-7 | hB5-2-8 |
| heavy chain hB5-VH3 | hB5-2-9 | hB5-2-10 | hB5-2-11 | hB5-2-12 |
| heavy chain hB5-VH4 | hB5-2-13 | hB5-2-14 | hB5-2-15 | hB5-2-16 |
| heavy chain hB5-VH5 | hB5-2-17 | hB5-2-18 | hB5-2-19 | hB5-2-20 |

### Example 4: Affinity test of humanized antibodies

A single concentration rapid measurement of affinity of the twenty hB5 humanized antibodies constructed in Example 3 was performed by Biacore 8K, so as to make a comparation between different humanized antibodies.

### Experimental protocol:

(1) Amino coupling of CM5 chip to Anti-Human Antibody protein.
(2) hB5 humanized antibodies flowing through the surface of the chip.
(3) High concentration TPBG-His antigen flowing through the surface of the chip.
(4) Affinity measurement.

### Experimental materials:

Antibody transfection system: 30 ml; Purification column: Protein A/G; The purity was above 95%.

### Experimental steps:

1) Ligand capturing
   - Cell containing supernatant was collected and centrifugation was performed at 4°C, 13000 rpm/min for 30 minutes. 2/3 of the supernatant was taken out and transferred to a new EP tube.
   - CM5 chip was separated into 8 Channels using a microfluidic chip system (IFC), with each Channel divided into 2 channels, in which channel 1 served as a reference channel, and channel 2 was used to capture the supernatant.
   - Flow rate was set to 10 µl/min, and the supernatant flew through for 200 seconds.
   - Over 95% of the antibodies captured were generally higher than 100 RU.
2) Antigen binding
   - TPBG-His protein was diluted with HBS-EP+buffer to 300 nM and flew through channel 1 and channel 2 at a flow rate of 30 µl/min for 120 seconds.
   - Glycine pH 1.7 flew through channel 1 and channel 2 at a flow rate of 30 µl/min for 40 seconds.
3) Response value of each antibody was obtained.
4) 1:1 Binding model of Bia-evaluation analysis software was used to calculate kinetic parameters and compare the affinity of each antibody.

### Experimental results:

The measurement results are shown in Table 3.

**Table 3. Statistics of kinetic parameters of cell containing supernatants and TPBG-His for different antibodies**

| **Supernatant** | **Primary antibody concentration (µg/ml)** | **ka (1/Ms)** | **kd (1/s)** | **KD (M)** | **Rmax (RU)** |
|---|---|---|---|---|---|
| **hB5-2-1** | 5 | 3.51E+05 | 3.88E-04 | 1.11E-09 | 107.7 |
| **hB5-2-2** | 5 | 2.23E+05 | 5.27E-04 | 2.36E-09 | 485.6 |
| **hB5-2-3** | stock solution | 3.64E+05 | 6.81E-04 | 1.87E-09 | 18.4 |
| **hB5-2-4** | 5 | 3.53E+05 | 2.98E-04 | 8.45E-10 | 272.1 |
| **hB5-2-5** | 5 | 3.37E+05 | 2.79E-04 | 8.27E-10 | 418.7 |
| **hB5-2-6** | 5 | 3.26E+05 | 4.97E-04 | 1.53E-09 | 327.7 |
| **hB5-2-7** | 5 | 3.93E+05 | 3.42E-04 | 8.69E-10 | 141.6 |
| **hB5-2-8** | 5 | 3.18E+05 | 3.02E-04 | 9.50E-10 | 448.8 |
| **hB5-2-9** | 5 | 2.99E+05 | 2.78E-04 | 9.27E-10 | 270.8 |
| **hB5-2-10** | 5 | 2.11E+05 | 4.87E-04 | 2.31E-09 | 593.6 |
| **hB5-2-11** | 5 | 3.10E+05 | 4.31E-04 | 1.39E-09 | 84.1 |
| **hB5-2-12** | 5 | 3.80E+05 | 2.98E-04 | 7.85E-10 | 322.6 |
| **hB5-2-13** | 5 | 4.34E+05 | 3.13E-04 | 7.21E-10 | 250.0 |
| **hB5-2-14** | 5 | 4.45E+05 | 7.74E-04 | 1.74E-09 | 248.4 |
| **hB5-2-15** | stock solution | 3.24E+05 | 6.32E-04 | 1.95E-09 | 98.5 |
| **hB5-2-16** | 5 | 5.47E+05 | 3.47E-04 | 6.34E-10 | 335.8 |
| **hB5-2-17** | 5 | 3.51E+05 | 3.17E-04 | 9.02E-10 | 326.8 |
| **hB5-2-18** | 5 | 2.94E+05 | 6.10E-04 | 2.07E-09 | 395.8 |
| **hB5-2-19** | stock solution | 3.96E+05 | 3.48E-04 | 8.80E-10 | 97.6 |
| **hB5-2-20** | 5 | 3.18E+05 | 3.02E-04 | 9.50E-10 | 421.8 |

It can be seen from Table 3 that all twenty hB5 humanized antibodies constructed in Example 3 exhibited good affinity, with affinity constants KD all reaching 10⁻⁹ M, and a considerable portion reaching 10⁻¹⁰ M.

### Example 5: Internalization rate test of humanized antibodies

In this example, the internalization rates of hB5-2-5, hB5-2-6, and hB5-2-17 in the humanized antibodies constructed in Example 3 were tested.

### Experimental protocol:

1) Cell collection: Old culture medium was removed, 0.25% trypsin digestion solution was added to digest the cells, and then DMEM culture medium was added to terminate the digestion. Cell suspension was collected, and centrifugation was performed at 800 rpm for 3 minutes.
2) Cells were resuspended in 4 ml of culture medium, and cell counting was performed: cell containing suspension was mixed well with 0.4% trypan blue in a 1:1 ratio, which was added to a cell counting plate, and the plate was placed in a Countstar cell counter to automatically count the number and viability of cells.
3) Cell containing suspension was divided into equal groups, with 1 × 10⁶ cells in the isotype control group and the negative control group, and 2 × 10⁶ cells in each experimental group.
4) Cells were subjected to centrifugation at 4 °C, 3500 rpm/min for 3 minutes, and the supernatant was carefully aspirated with a pipette tip.
5) Incubation with primary antibody: 100 µl of mouse IgG (at a concentration of 10 µg/ml) was added to the isotype control group, 100 µl of PBS was added to the blank control group, and 100 µl of antibody solution with a final concentration of 10 µg/mL was added to the experimental group. The groups were marked, the cells were resuspended and then incubated on ice for 30-40 minutes. Then, the cells were resuspended in 500 µl PBS buffer, followed by centrifugation at 4 °C, 3500 rpm/min for 3 minutes, and washing twice repeatedly;
6) After resuspension of cells in 500 µl PBS, the cells in the experimental group were divided into two groups, in which the non-internalization group was incubated on ice or at 4 °C for 3 hours, and the internalization group was incubated at 37 °C for 3 hours, while the isotype and negative control groups were temporarily incubated at 4 °C; During this period, the tube wall was gently tapped every 10 minutes to avoid cell precipitation.
7) After incubation, centrifugation was performed at 3500 rpm and 4 °C for 3 minutes, after which the supernatant was discarded, and the cells were washed twice;
8) Incubation with secondary antibody: the secondary antibody (FITC-goat-antimouse IgG antibody/FITC-goat-anti-human IgG antibody) was diluted with PBS at a ratio of 1:200, 100 µl of secondary antibody was added to each EP tube for resuspension, and then incubated on ice at 4 °C for 40 minutes.
9) After incubation, 500 µl of PBS buffer was added to each group, and the cells were resuspended and blown twice. Then, the cells in each group were centrifuged at 3500 rpm and 4 °C for 3 minutes, and the supernatant was aspirated. This process was repeated 2-3 times;
10) 300 µl PBS buffer was added to resuspend each group of cells, which was aspirated and added to a flow cytometer by a pipette, and allowed to be tested on the machine.
11) On a flow cytometer, all parameters was set, and the cells in the blank control group were used as control after adjustment. Then, the cell samples of each group were tested subsequently.

### Experimental results:

Cell: CHO-5T4+; Antibody gradient: 0.1, 0.5, 1 µg/ml; Internalization time: 3 hours.

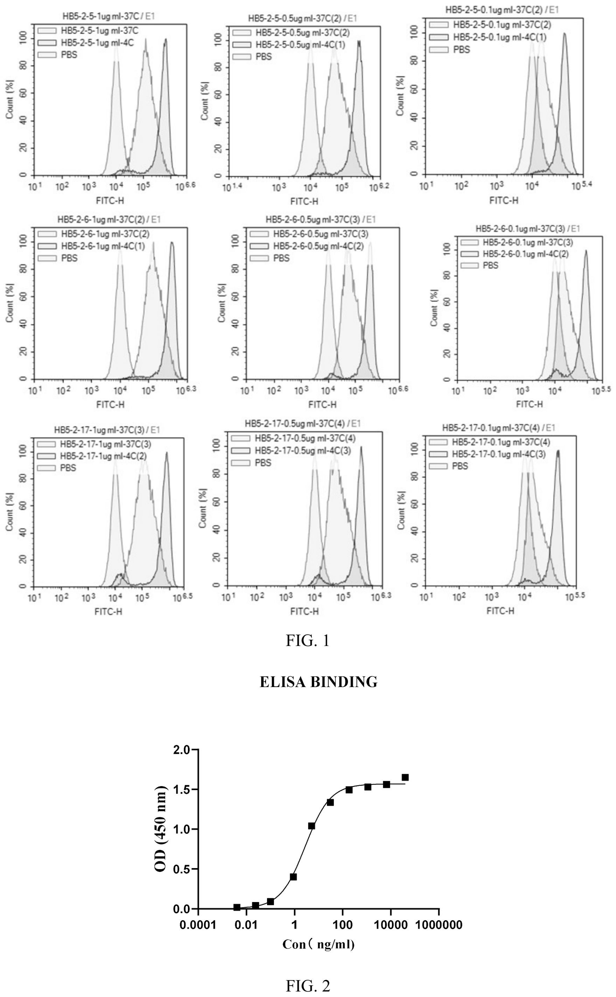

The measurement results are shown in Figure 1 and Table 4.

**Table 4. Internalization rate of humanized antibodies**

| Sample Name | Concentration | | |
|---|---|---|---|
| | 0.1 µg/ml | 0.5 µg/ml | 1 µg/ml |
| hB5-2-5 | 65.99% | 70.27% | 72.38% |
| hB5-2-6 | 66.13% | 72.08% | 67.03% |
| hB5-2-17 | 70.87% | 73.24% | 73% |

It can be seen that the internalization rates of the three humanized antibodies were around 60%-70%, showing a high internalization rate, among which hB5-2-17 performed the best.

### Example 6: Performance test of humanized antibody hB5-2-10

In this example, various properties of humanized antibody hB5-2-10 were tested.

### (1) Affinity - ELISA/Biacore

### ELISA detection of affinity between hB5-2-10 and TPBG-His

### Experimental protocol:

1) Coating: TPBG-His was diluted to 1 µg/ml with 50 mM carbonate buffer (pH 9.6). Then, it was added 50 µl per well to an ELISA plate. A sealing film was added, followed by storing overnight in a refrigerator at 4 °C.
2) Plate washing: the sealing film was removed and the plate was placed in the plate washing machine. 300 µl of 1 × PBST was added to each well, followed by shaking for 20 seconds, and soaking for 10 seconds. The plate was washed twice. The ELISA plate was taken out and gently pated on absorbent paper until there was no liquid residue or bubbles in the wells.
3) Sealing: 200 µl of 1% BSA was added to each well, followed by incubation at 37 °C for 1 hour.
4) Plate washing: the sealing film was removed and the plate was placed in the plate washing machine. 300 µl of 1 × PBST was added to each well, followed by shaking for 20 seconds, and soaking for 10 seconds. The plate was washed twice. The ELISA plate was taken out and gently pated on absorbent paper until there was no liquid residue or bubbles in the wells.
5) Incubation with primary antibody: hB5-2-10 was diluted with 0.1% BSA to a concentration of 40000 ng/ml, then it was diluted in a 6-fold gradient for a total of 10 concentration gradients. It was then added to the plate at 100 µl per well, with 2 replicates, and incubated at 37 °C for 1 hour.
6) Plate washing: the sealing film was removed and the plate was placed in the plate washing machine. 300 µl of 1 × PBST was added to each well, followed by shaking for 20 seconds, and soaking for 10 seconds. The plate was washed four times. The ELISA plate was taken out and gently pated on absorbent paper until there was no liquid residue or bubbles in the wells.
7) Incubation with secondary antibody: Goat-Anti-human IgG Fc (HRP) was diluted with 0.1% BSA at a dilution ratio of 1:10000. It was then added to the plate at 100 µl per well, and incubated at 37 °C for 1 hour.
8) Plate washing: the sealing film was removed and the plate was placed in the plate washing machine. 300 µl of 1 × PBST was added to each well, followed by shaking for 20 seconds, and soaking for 10 seconds. The plate was washed three times. The ELISA plate was taken out and gently pated on absorbent paper until there was no liquid residue or bubbles in the wells.
9) Color development: 100 µl of TMB color developing agent was added to each well, followed by incubation at 37 °C for 10 minutes.
10) Adding of termination solution: 100 µl of 0.7 M oxalic acid solution was added to each well to terminate the reaction.
11) Measuring of OD value: an ELISA reader was used to measure the absorbance at a wavelength of 450 nm.

### Experimental results:

The ELISA results are shown in Figure 2. The EC₅₀ of antibody hB5-2-10 binding to TPBG-His was 2.801 ng/ml.

### Biacore detection of affinity between hB5-2-10 and TPBG-His

### Experimental protocol:

1) CM5 chip was separated into 8 Channels using a microfluidic chip system (IFC), with each Channel divided into 2 channels, in which channel 1 served as a reference channel, and channel 2 was used to capture the ligand.
2) pH pre-enrichment: NaAC buffer (pH 4.5/5.0) was used to connect the antigen (TPBG-His) to the CM5 chip at a concentration of 10 µg/mL. After determining the appropriate antibody dilution solution and antibody concentration, the antibody was directly coupled to the CM5 chip. Flow rate was set to 10 µl/min, contact time was 2 min, dissociation time was 0, and 50 mM NaOH was used as the regeneration reagent. The pH value for the coupling was determined based on the results.
3) Coupling of ligand: TPBG-His antigen was diluted with 10 mM acetate coupling reagent with a predetermined pH value to a suitable concentration determined by pre-enrichment. Then the CM5 chip was activated with (20%)EDC+(80%)NHS, at a reaction time of 30 s and flow rate of 10 µ l/min, and the coupled ligand was blocked with ethanolamine at a reaction time of 420 s, so as to complete the ligand coupling process.
4) Analyte binding

The antibody as an analyte was diluted in different concentration gradients, and then flew through channel 1 and channel 2 sequentially at a flow rate of 30 µl/min for 120 seconds, with a dissociation time set at 600 seconds.

Regeneration buffer glycine pH 1.5 flew through channel 1 and channel 2 at a flow rate of 30 µl/min for 30 seconds.

5) Response value for each concentration of analyte was obtained. 1:1 Binding model of Bia-evaluation analysis software was used to calculate kinetic parameters and compare the affinity of each antibody.

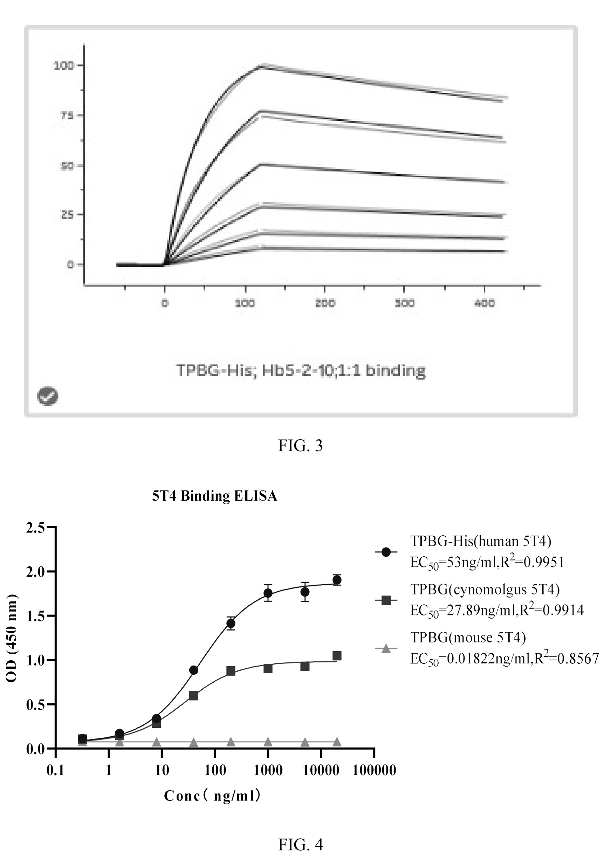

The Biacore results are shown in Figure 3 and Table 5.

**Table 5. Biacore measurement results of antibody hB5-2-10**

| Ligand | ka (1/Ms) | kd (1/s) | KD (M) |
|---|---|---|---|
| TPBG-His | 2.15E+05 | 6.27E-04 | 2.92E-09 |

### (2) Cross reactivity - ELISA

### Experimental protocol:

1) Coating: 3 different species of TPBG-His were diluted to 1 µg/ml with 1 × PBS buffer. Then, it was added 50 µl per well to an ELISA plate. A sealing film was added, followed by storing overnight in a refrigerator at 4 °C.
2) Plate washing: the sealing film was removed and the plate was placed in the plate washing machine. 300 µl of 1 × PBST was added to each well, followed by shaking for 3 seconds, and soaking for 40 seconds. The plate was washed twice. The ELISA plate was taken out and gently pated on absorbent paper until there was no liquid residue or bubbles in the wells.
3) Sealing: 250 µl of 1% BSA was added to each well, followed by incubation at 37 °C for 1 hour.
4) Plate washing: the sealing film was removed and the plate was placed in the plate washing machine. 300 µl of 1 × PBST was added to each well, followed by shaking for 3 seconds, and soaking for 40 seconds. The plate was washed twice. The ELISA plate was taken out and gently pated on absorbent paper until there was no liquid residue or bubbles in the wells.
5) Incubation with primary antibody: hB5-2-10 was diluted with 0.1% BSA to concentrations of 20000 ng/ml, 5000 ng/ml, 1000 ng/ml, 200 ng/ml, 40 ng/ml, 8 ng/ml, 1.6 ng/ml, and 0.32 ng/ml, and a 0 concentration point of only 1% BSA was set. It was then added to the plate at 100 µl per well, with 2 replicates, and incubated at 37 °C for 1 hour.
6) Plate washing: the sealing film was removed and the plate was placed in the plate washing machine. 300 µl of 1 × PBST was added to each well, followed by shaking for 3 seconds, and soaking for 50 seconds. The plate was washed three times. The ELISA plate was taken out and gently pated on absorbent paper until there was no liquid residue or bubbles in the wells.
7) Incubation with secondary antibody: Goat-Anti-human IgG Fc (HRP) was diluted with 0.1% BSA at a dilution ratio of 1:7000. It was then added to the plate at 100 µl per well, and incubated at 37 °C for 45 minutes.
8) Plate washing: the sealing film was removed and the plate was placed in the plate washing machine. 300 µl of 1 × PBST was added to each well, followed by shaking for 3 seconds, and soaking for 50 seconds. The plate was washed three times. The ELISA plate was taken out and gently pated on absorbent paper until there was no liquid residue or bubbles in the wells.
9) Color development: 100 µl of TMB color developing agent was added to each well, followed by incubation at 37 °C for 10 minutes.
10) Adding of termination solution: 100 µl of 2 M H₂SO₄ termination solution was added to each well to terminate the reaction.
11) Measuring of OD value: an ELISA reader was used to measure the absorbance at a wavelength of 450 nm.

### Experimental results:

The results are shown in Figure 4, which indicates that antibody hB5-2-10 bound to human/monkey 5T4 antigen with similar binding activity and did not bind to mouse 5T4 antigen.

### (3) Cell binding - FACS

### Experimental protocol:

1) 3 × 10⁵ cells were taken out, and subjected to centrifugation at 3500 rpm for 5 minutes. Then, the supernatant was discarded, and 500 µl of 1 × PBS buffer was added for washing. Centrifugation was performed at 3500 rpm for 5 minutes, and then the supernatant was discarded. This process was repeated twice.
2) The test sample was diluted with 1 × PBS buffer to a starting concentration of 20 µg/ml, then it was diluted in a 5-fold gradient. 100 µl/sample was added, followed by incubation at 2-8 °C for 1 hour.
3) 100 µl/sample of each sample was added, followed by incubation on ice or at 2-8 °C for 1 hour. During this period, the cells were scattered every 15 minutes.
4) After incubation was completed, 500 µl 1 × PBS buffer was added for washing, followed by centrifugation at 3500 rpm for 5 minutes, and then the supernatant was discarded. This process was repeated three times.
5) FITC-anti human IgG secondary antibody was diluted at 1:100. 100 µl/sample was added, followed by incubation on ice for 40 minutes. During this period, the cells were scattered every 10 minutes.
6) After incubation was completed, 500 µl 1 × PBS buffer was added for washing, followed by centrifugation at 3500 rpm for 5 minutes, and then the supernatant was discarded. This process was repeated three times.
7) Finally, resuspension was performed with 300 µl of 1 × PBS buffer, followed by detection on the machine.

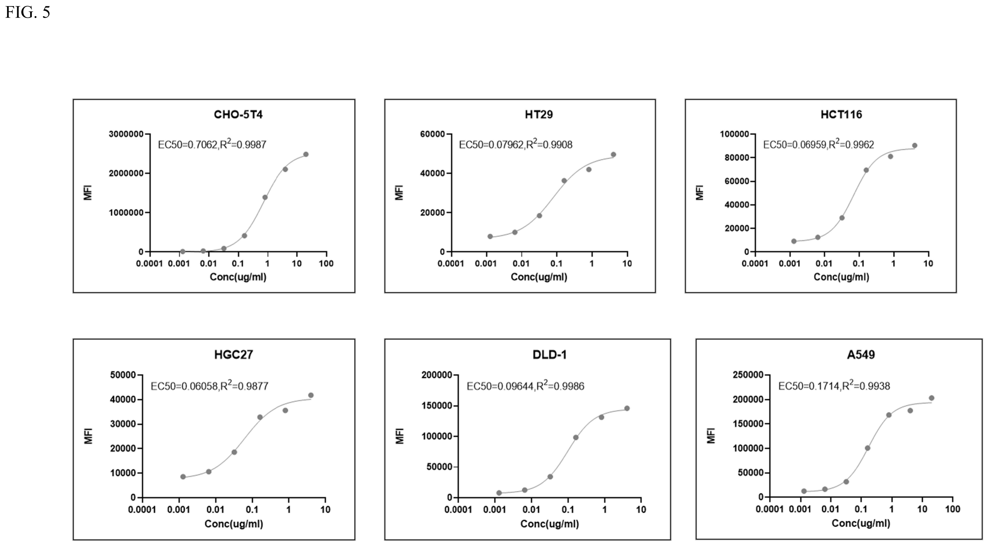

The results are shown in Figure 5, which indicates that antibody hB5-2-10 exhibited good cell binding activity.

### (4) Internalization - FACS

### Experimental protocol:

1) Cells were collected and subjected to detection for viability, with 3 × 10⁵ cells taken for each test;
2) Incubation with primary antibody: hB5-2-10 was diluted to 2 µg/ml, then 100 µl/sample was added; A negative control PBS group was provided. Incubation was performed at 2-8 °C for 1 hour, followed by washing once with 1 × PBS buffer.
3) Incubation for internalization: Each group of cells was divided into two equal parts, one part was incubated on ice as the non-internalization group, and the other part was incubated in a 37 °C constant temperature incubator as the internalization group. Both groups were incubated simultaneously for 4 hours. After incubation, gentle blowing was performed and the cells were aspirated, and 500 µl of 1 × PBS buffer was added for washing, followed by centrifugation at 3500 rpm for 5 minutes, and then the supernatant was discarded.
4) Incubation with secondary antibody: All groups were taken out simultaneously and secondary antibody FITC-goat-anti-human IgG was added, followed by dilution at 1:100. 100 µl/sample was added, and incubation was performed at 2-8 °C for 30 minutes. After incubation was completed, washing was performed once.
5) Resuspension was performed with 300 µl of 1 × PBS buffer, followed by detection on the machine.

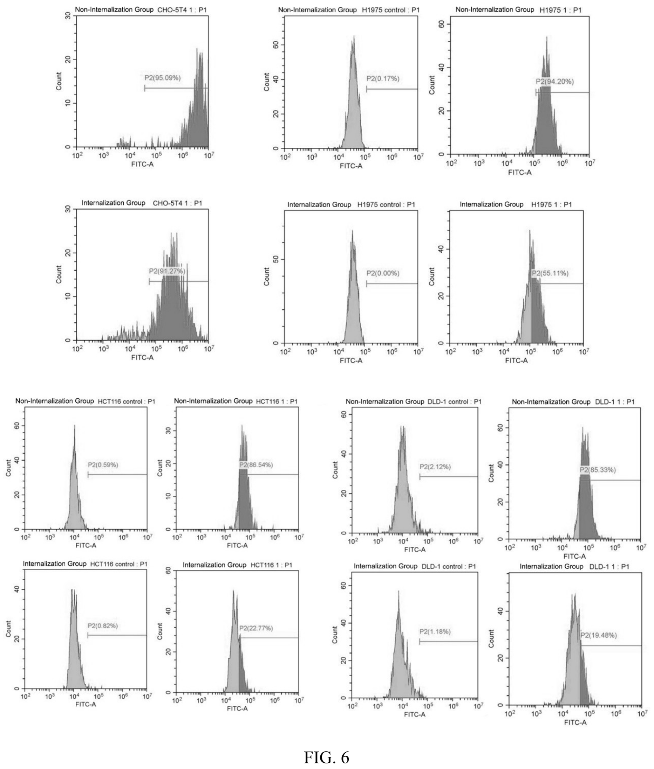

The results are shown in Figure 6 and Table 6, where internalization rate = (non-internalization group - internalization group)/non-internalization group. It can be seen that antibody hB5-2-10 exhibited a high internalization rate.

**Table 6. Internalization rate of antibody hB5-2-10**

| cells | **CHO-5T4** | | | **H1975** | | |
|---|---|---|---|---|---|---|
| MFI | Non-internalizat ion group | Internalizat ion group | Internalizat ion rate | Non-internalizat ion group | Internalizat ion group | Internalizat ion rate |
| Control group | 12144.2 | 19161 | | 39815.4 | 39390.8 | |
| Experim ent group | 4111599.3 | 646607.5 | | 285465.8 | 152371.3 | |
| Experim ent group - Control group | 4099455.1 | 627446.5 | 84.69% | 245650.4 | 112980.5 | 54.01% |

| cells | **HCT116** | | | **DLD-1** | | |
|---|---|---|---|---|---|---|
| | Non-internalizat ion group | Internalizat ion group | Internalizat ion rate | Non-internalizat ion group | Internalizat ion group | Internalizat ion rate |
| Control group | 11893.6 | 12139 | | 13669.5 | 11080.4 | |
| Experim ent group | 63056 | 30587.6 | | 85741.2 | 35046.4 | |
| Experim ent group - Control group | 51162.4 | 18448.6 | 63.94% | 72071.7 | 23966 | 66.75% |

It should be understood that although the present invention has been specifically disclosed through preferred embodiments and optional features, those skilled in the art may modify, improve, and vary the present invention disclosed herein, and these modifications, improvements, and variations are considered within the scope of the present invention. The materials, methods, and embodiments provided herein are representative and exemplary of preferred embodiments and are not intended to limit the scope of the invention.

## Claims

1. A monoclonal antibody binding to 5T4 or an antigen binding fragment thereof, comprising a light chain variable region and a heavy chain variable region, wherein the light chain variable region comprises LCDR1, LCDR2, and LCDR3, and the heavy chain variable region comprises HCDR1, HCDR2, and HCDR3, wherein:
the LCDR1, LCDR2, and LCDR3 comprise or consist of respective CDR1, CDR2, CDR3 of a light chain variable region with an amino acid sequence set forth in SEQ ID NO: 7, or equivalent variants thereof, and
the HCDR1, HCDR2, and HCDR3 comprise or consist of respective CDR1, CDR2, CDR3 of a heavy chain variable region with an amino acid sequence set forth in SEQ ID NO: 8, or equivalent variants thereof.

2. The monoclonal antibody or an antigen binding fragment thereof of claim 1, wherein the LCDR1, LCDR2, LCDR3, HCDR1, HCDR2, and HCDR3 are defined according to any one of the definition schemes selected from the group consisting of IMGT, Kabat, Chothia, Contact, or Martin definition scheme.

3. The monoclonal antibody or an antigen binding fragment thereof of claim 1, wherein:
the LCDR1 comprises or consists of a sequence set forth in SEQ ID NO: 1 or an equivalent variant thereof, the LCDR2 comprises or consists of a sequence set forth in SEQ ID NO: 2 or an equivalent variant thereof, and the LCDR3 comprises or consists of a sequence set forth in SEQ ID NO: 3 or an equivalent variant thereof, and
the HCDR1 comprises or consists of a sequence set forth in SEQ ID NO: 4 or an equivalent variant thereof, the HCDR2 comprises or consists of a sequence set forth in SEQ ID NO: 5 or an equivalent variant thereof, and the HCDR3 comprises or consists of a sequence set forth in SEQ ID NO: 6 or an equivalent variant thereof.

4. The monoclonal antibody or an antigen binding fragment thereof of claim 1, wherein the antibody is a humanized antibody or a chimeric antibody.

5. The monoclonal antibody or an antigen binding fragment thereof of claim 1, wherein:
the light chain variable region comprises or consists of a sequence set forth in SEQ ID NO: 7 or an equivalent variant thereof, and
the heavy chain variable region comprises or consists of a sequence set forth in SEQ ID NO: 8 or an equivalent variant thereof.

6. The monoclonal antibody or an antigen binding fragment thereof of claim 1, wherein:
the light chain variable region comprises or consists of any sequence selected from the group consisting of SEQ ID NO: 9, SEQ ID NO: 10, SEQ ID NO: 11, and SEQ ID NO: 12, and an equivalent variant of each thereof, and
the heavy chain variable region comprises or consists of any sequence selected from the group consisting of SEQ ID NO: 13, SEQ ID NO: 14, SEQ ID NO: 15, SEQ ID NO: 16, and SEQ ID NO: 17, and an equivalent variant of each thereof.

7. A polynucleotide comprising a polynucleotide encoding the antibody of any one of claims 1 to 6.

8. A vector comprising the polynucleotide of claim 7.

9. A non-human host cell, comprising the vector of claim 8.

10. A pharmaceutical composition comprising a therapeutic effective amount of the antibody of any one of claims 1 to 6, the polynucleotide of claim 7, the vector of claim 8, or the host cell of claim 9, and a pharmaceutically acceptable carrier.
